# EUROPEAN PATENT APPLICATION

(11) **EP 2 952 211 A1**
(43) Date of publication of application: **09.12.2015**
(21) Application number: 14745511.7
(22) Date of filing: 22.01.2014
(51) Int. Cl.: A61K 49/00, A61K 9/127

(54) **CONTRAST MEDIUM, AND PREPARATION METHOD AND PREPARATION KIT THEREFOR**

(30) Priority: 31.01.2013 JP 2013016610
(71) Applicant: Olympus Corporation, Shibuya-ku Tokyo 151-0072 (JP); The University of Tokyo, Tokyo 113-8654 (JP)
(72) Inventor: MURAKAMI, Miyuki, Tokyo 151-0072 (JP); YAMAGUCHI, Satoshi, Tokyo 113-8654 (JP); NAGAMUNE, Teruyuki, Tokyo 113-8654 (JP); IIJIMA, Mariko, Tokyo 113-8654 (JP); SHIBASAKI, Yoshikazu, Tokyo 113-8654 (JP)
(74) Representative: Schicker, Silvia
(86) International application number: PCT/JP2014/051311
(87) International publication number: WO 2014/119455

(57) **Abstract**

Provided is a contrast medium (1) comprising at least one molecular probe (3 to 5) and at least one optical probe (6) on a surface of a substrate (2) having an ultrasound imaging function. More specifically, a contrast medium (1) having sensitivity to not only ultrasound but also light excellent in real-time imaging and resolution and having a size sufficient to travel into tissues through blood vessel can be provided by labelling an ultrasound imageable substrate (2) with a molecular probe (3 to 5) and an optical probe (6).

## Description

### {Technical Field}

The present invention relates to a contrast medium, and a method and kit for preparing the contrast medium.

### {Background Art}

Diagnostic imaging modality such as X-ray CT, MRI and ultrasonic diagnostic equipment has long been used as an essential tool at medical front. In these tools, imaging is made based on difference in *in-vivo* CT values, spin relaxation time and acoustic impedance. Since difference in these physical properties exclusively reflects a structure (figure) of a living body, the modality is called "form imaging".

In contrast, imaging of a site of the same structural tissue but different in function is called "functional imaging". Particularly in the functional imaging, visualizing the present states of molecules, such as proteins, constituting a living organism, is often called "molecular imaging". The molecular imaging is expected to be used in understanding life phenomena such as development and differentiation and applied to diagnosing and treating diseases. Because of this, the molecular imaging is a research field currently most receiving attention.

In the molecular imaging, a "molecular probe" is frequently used, which is a substance having structural selectivity to molecules constituting a living organism. In this case, to the molecular probe, a detectable structure by any physical means is added and the distribution of the molecular probe in a body is visualized. Attempt to use such molecular imaging in early diagnosis has been accelerated in recent years.

In an attempt to use modality such as MRI and ultrasonic diagnostic equipment, which has been previously used in form-imaging, in the molecular imaging, research and development are conducted. Ultrasonic diagnostic equipment has the following features that other modality tools do not provide: (1) real time analysis can be excellently made, (2) because of small size, use in operation rooms is rarely restricted and (3) not only used as a diagnosis tool but also used as a treatment tool. For the reason, the ultrasonic diagnostic equipment is further expected as a diagnosis-therapy integrated tool that can be used other than large hospitals.

To realize molecular imaging for "early diagnosis for a disease", a site-specific oil-in-water emulsion using a liquid microbubble-precursor is conventionally known as an ultrasound contrast medium (for example, see Patent Literature 1).

The microbubble-precursor is a chemical substance that is easily gasified and can be stably present in a liquid state in the emulsion. A contrast medium, which produces micro air bubbles when ultrasound energy is applied thereto and has a size sufficient to travel from a blood vessel to a tissue, is proposed.

The microbubble precursor is administered into a living body in the form of nano-size liquid drops; however, liquid drops cause phase change by ultrasound irradiation to generate microbubbles. The microbubble precursor is, i.e., a phase-change ultrasound contrast medium. Owing to the ability of forming micro air bubbles by application of ultrasound energy, the contrast medium works in a site-specific manner. In addition, if a molecular probe such as antibody and ligand which selectively binds to a molecule constituting a living body is added, tissue selectivity can be provided.

### {Citation List}

### {Patent Literature}

{PTL 1}
Publication of Japanese Patent No. 3016592

### {Summary of Invention}

### {Technical Problem}

However, the ultrasound contrast medium formed of a microbubble precursor disclosed in Patent Literature 1 has a disadvantage in that the presence of the contrast medium in and out a cell cannot be determined; in particular, whether it is incorporated in a cell or not cannot be determined.

The present invention was made in consideration of the aforementioned circumstances. An object of the invention is to provide a contrast medium whose uptake within a cell can be easily determined, a method and kit for preparing the contrast medium.

### {Solution to Problem}

To attain the above object, the present invention will provide the following means.

According to one aspect of the present invention, there is provided a contrast medium comprising at least one targeting molecular probe and at least one optical probe on a surface of a substrate having an ultrasound imaging function.

In the aspect, the contrast medium may have a first molecular probe for conjugation, an optical probe labeled with a second molecular probe and a third molecular probe for targeting capable of binding to a cell or a tissue in a disease-specific manner.

In the aspect, the third molecular probe may be labeled with the first molecular probe.

In the aspect, the second molecular probe and the third molecular probe may be contained in a molar ratio of 1:1.

In the aspect, the substrate may be a capsule-type contrast medium comprising a contrast substance having an imaging function included in an outer coat formed of lipid membrane.

In the aspect, the contrast substance may be a water insoluble substance vaporizable by ultrasound irradiation.

In the aspect, the contrast substance may be a gaseous material.

According to another aspect of the present invention, there is provided a method for preparing the contrast medium, comprising: a first step of producing a first solution containing the substrate of which the surface is labeled with the first molecular probe; a second step of producing a second solution containing the second molecular probe labeled with the optical probe; a third step of producing a third solution containing the third molecular probe labeled with the first molecular probe; a fourth step of producing a fourth solution by mixing the second solution produced in the second step and the third solution produced in the third step; a fifth step of mixing and reacting the first solution produced in the first step with the solution produced in the fourth step with the first solution produced in the first step; and a sixth step of centrifuging the solution mixture produced in the fifth step.

In the aspect, the second molecular probe contained in the second solution and the third molecular probe contained in the third solution to be mixed in the fourth step may be present in a molar ratio of 1:1.

In the aspect, in the fifth step, a 10-fold or more dilution of the first solution produced in the first step may be mixed.

According to another aspect of the present invention, there is provided a kit for preparing the contrast medium as mentioned above, comprising: a plurality of storage containers separately storing a first solution containing the substrate of which a surface is labeled with the first molecular probe, a second solution containing the second molecular probe labeled with the optical probe, and a third solution containing the third molecular probe labeled with the first molecular probe; and openable and closable channels connected with the storage containers.

### {Advantageous Effects of Invention}

According to the present invention, an effect of easily determining whether a contrast medium is incorporated within a cell can be exerted.

### {Brief Description of Drawings}

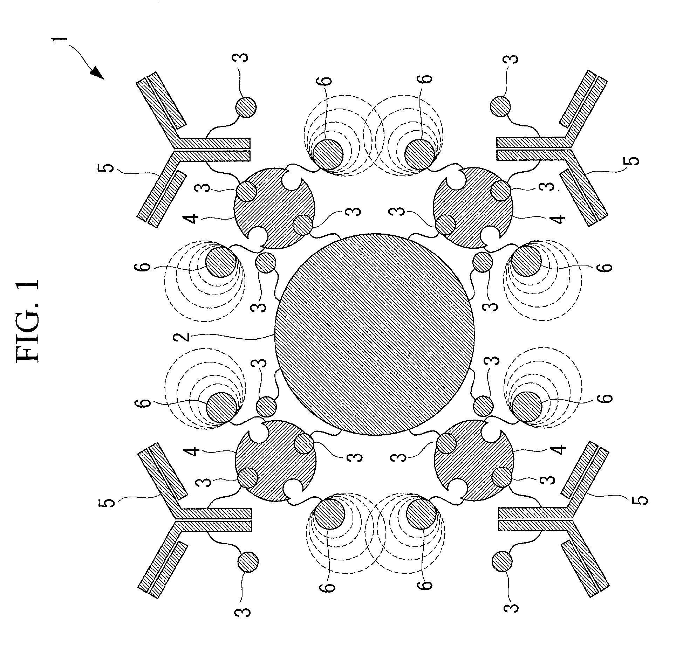

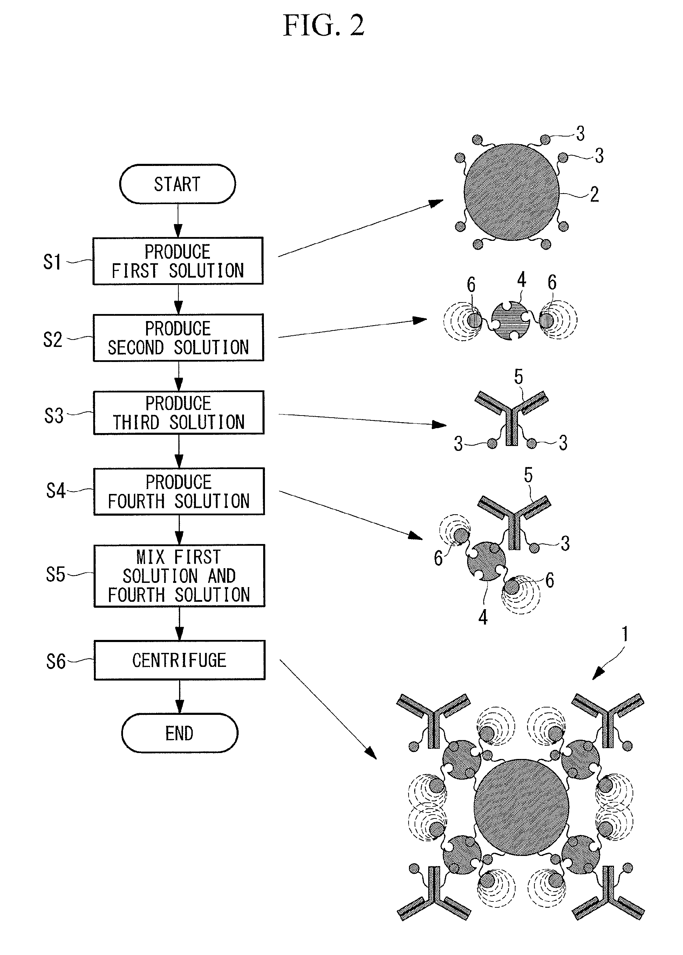

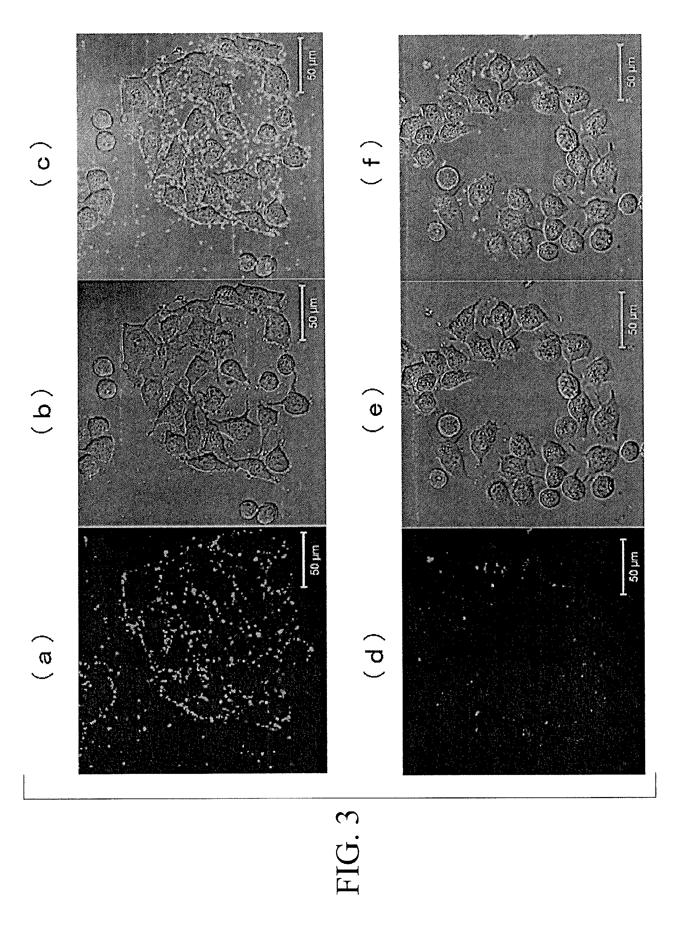

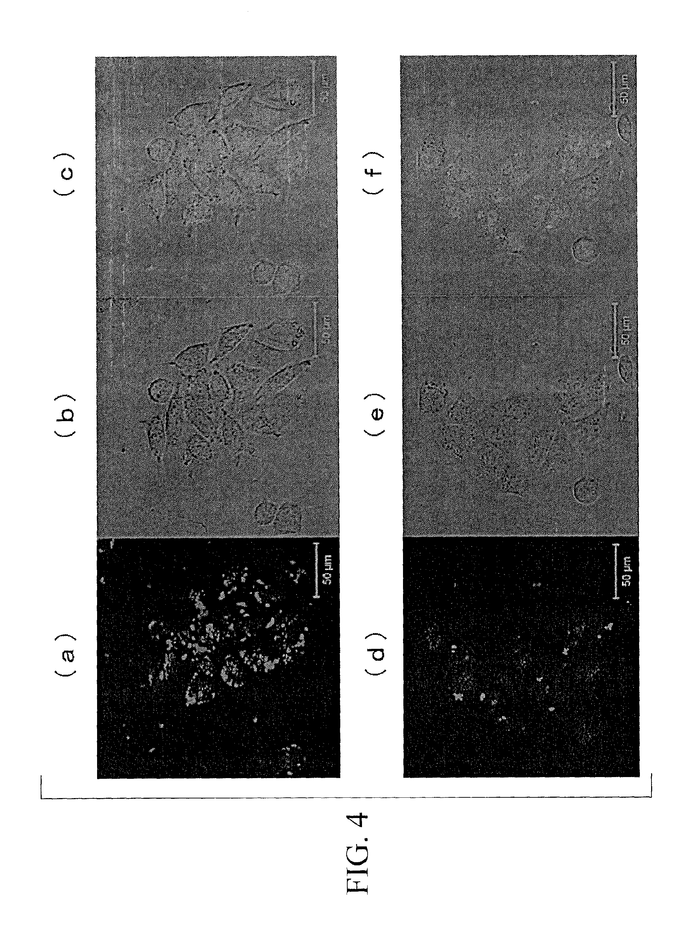

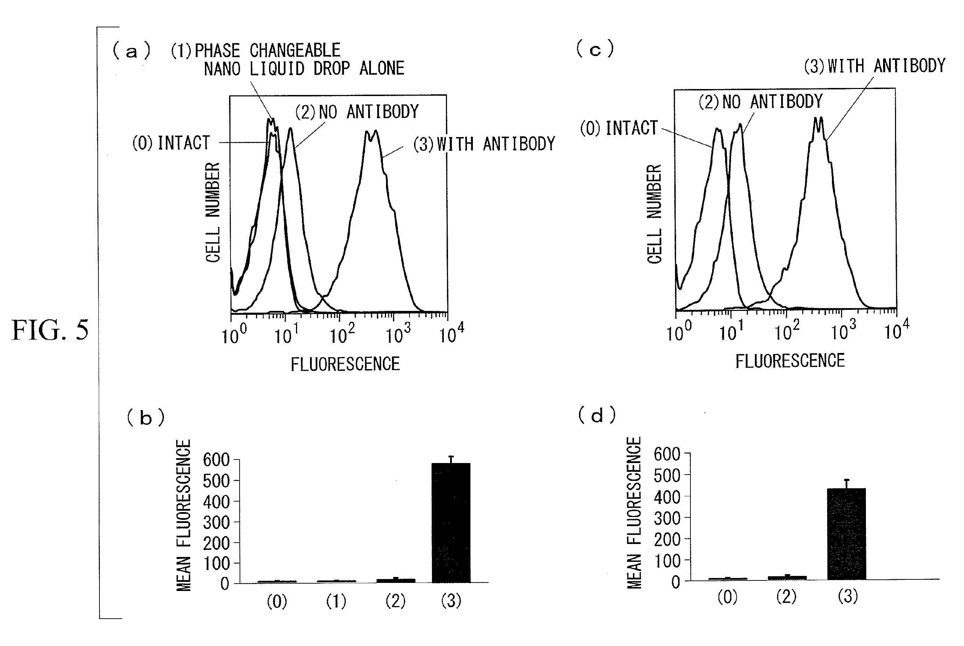

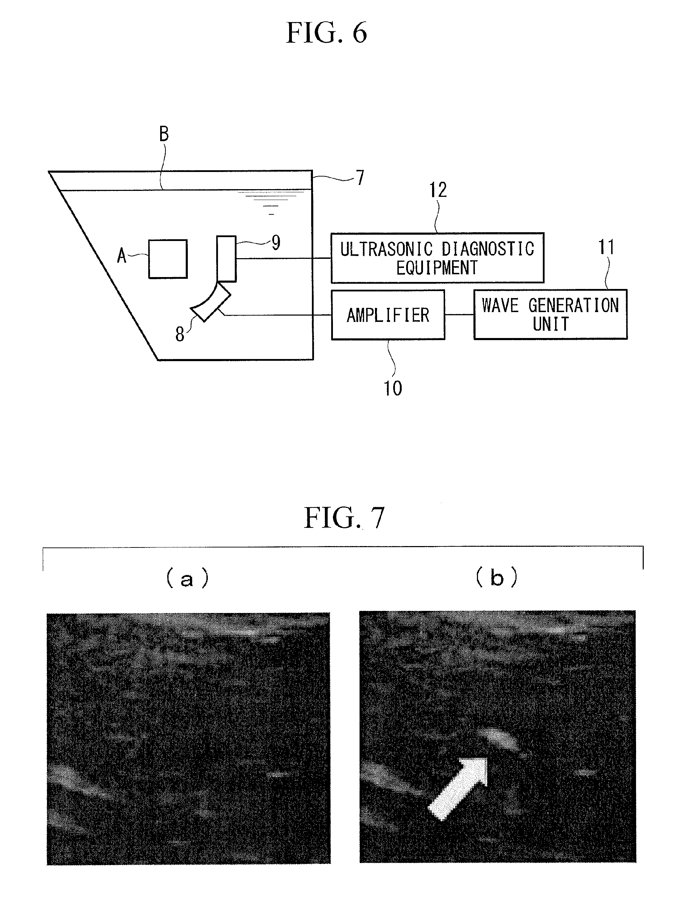

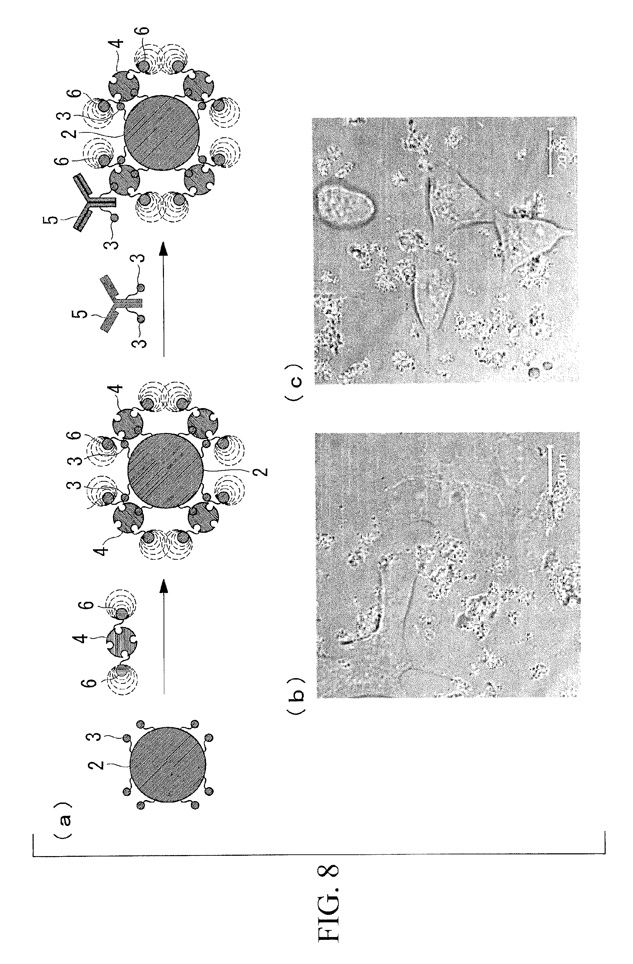

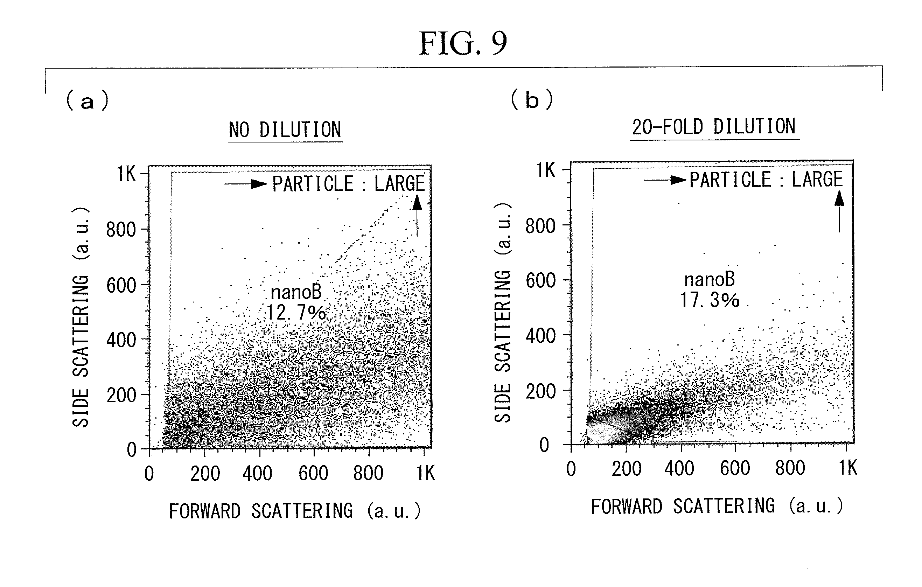

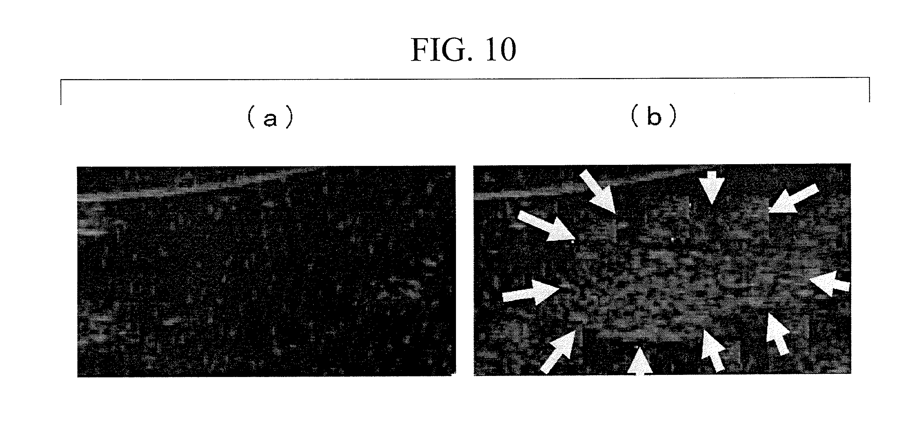

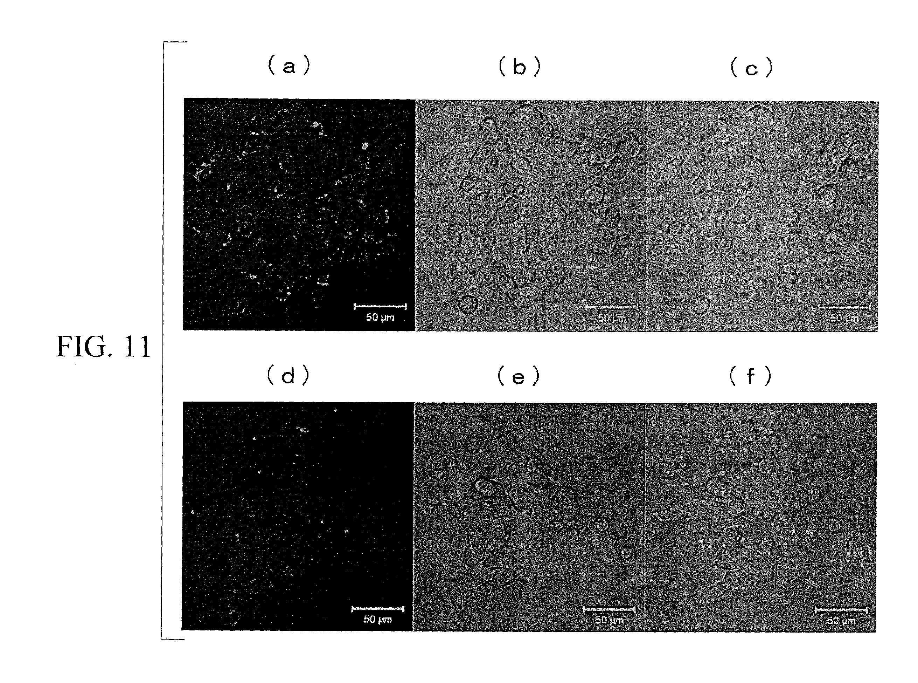

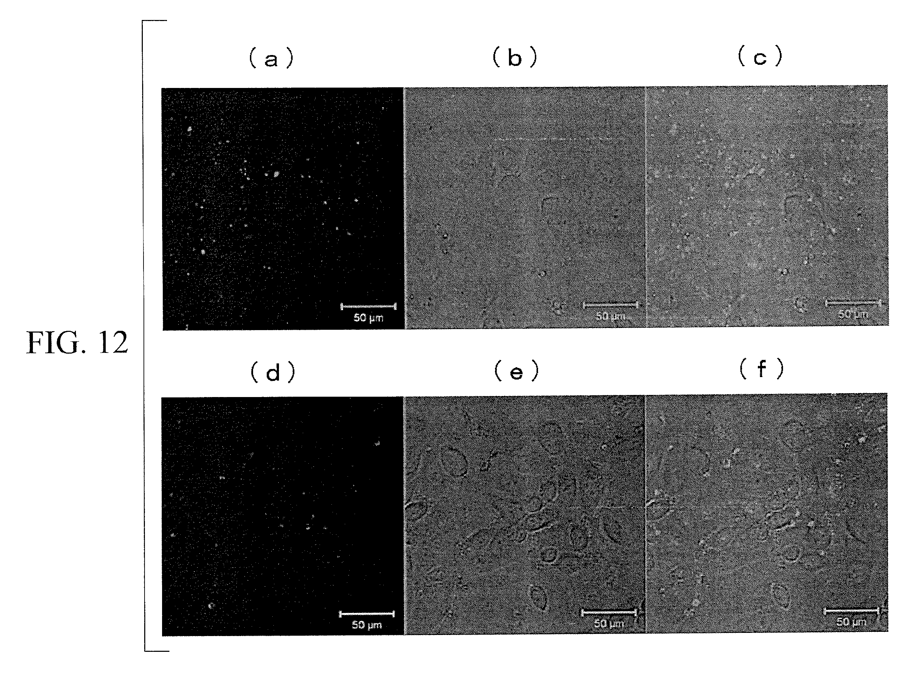

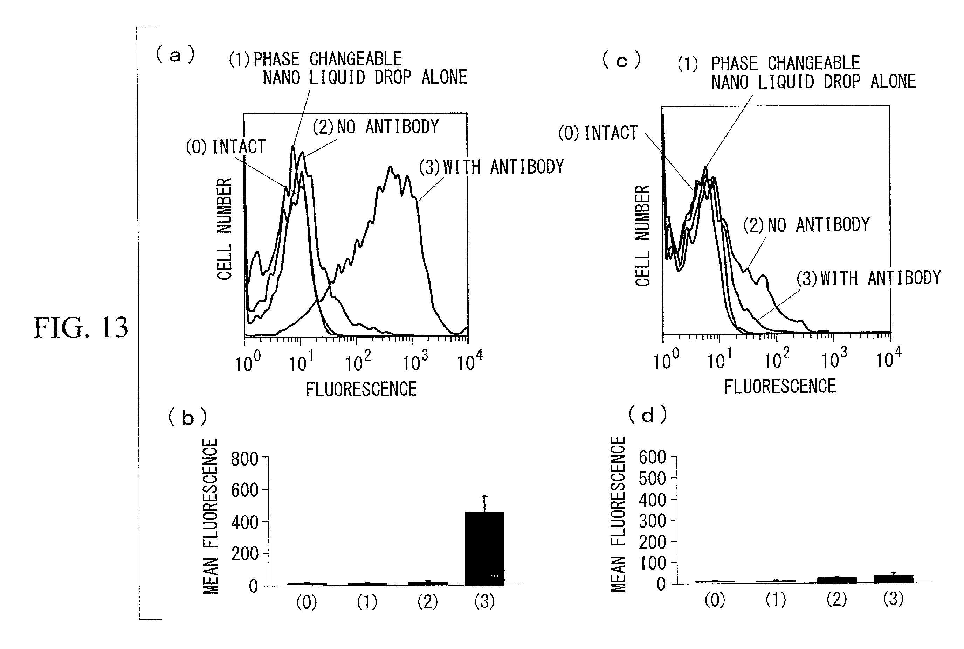

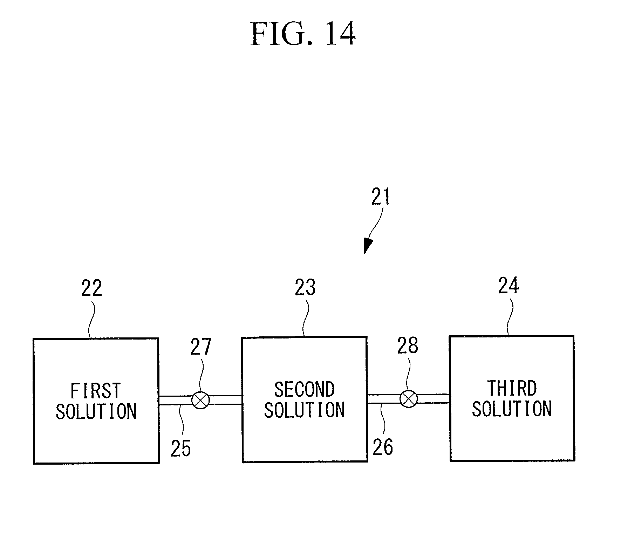

{Fig. 1}
   The figure is a view schematically showing the molecular structure of a contrast medium according to a first embodiment of the present invention.
{Fig. 2}
   The figure shows a flowchart of a method for preparing the contrast medium of Fig. 1.
{Fig. 3}
   The figure shows respectively (a) a confocal fluorescence microscope image, (b) a bright-field image and (c) a superimposed image all showing specific binding ability of the contrast medium of Fig. 1 to cancer cells highly expressing CCA1 (30 minutes after the contrast medium is sprinkled on the cells before culture); and (d) a confocal fluorescence microscope image, (e) a bright-field image and (f) a superimposed image all showing a contrast medium of Comparative Example having no CCA1 antibody.
{Fig. 4}
   The figure shows respectively (a) a confocal fluorescence microscope image, (b) a bright-field image and (c) a superimposed image all showing specific uptake of the contrast medium of Fig. 1 by cancer cells highly expressing CCA1 (six hours after culture); and (d) a confocal fluorescence microscope image, (e) a bright-field image and (f) a superimposed image all showing a contrast medium of Comparative Example having no CCA1 antibody.
{Fig. 5}
   The figure shows flow-cytometric analysis results showing specific uptake of the contrast medium of Fig. 1 by cancer cells (six hours after culture): (a) relationship between amount of fluorescence and the number of cells due to uptake of phase changeable nano liquid drops by cancer cells highly expressing CCA1, (b) the mean fluorescence of each of the cases in graph (a), (c) relationship between amount of fluorescence and the number of cells due to uptake of phase changeable nano liquid drops containing a different component and (d) the mean fluorescence of each of the cases in graph (c).
{Fig. 6}
   The figure shows the experiment system for irradiation of a tissue to which the contrast medium of Fig. 1 is administered with ultrasound and imaging.
{Fig. 7}
   The figure shows ultrasound images (a) before irradiation and (b) after irradiation with ultrasound at an intensity required for vaporization.
{Fig. 8}
   The figure shows respectively confocal microscope analysis results of the contrast medium of Comparative Example produced in accordance with different order of steps from that of the preparation method shown in Fig. 2: (a) flowchart of the preparation method, (b) confocal microscope analysis results of a contrast medium having no CCA1 antibody and produced by skipping the second step of the method (a), (c) confocal microscope analysis results of a contrast medium having CCA1 antibody and produced by the method including the second step.
{Fig. 9}
   The figure shows respectively (a) scattering of an undiluted case and (b) scattering of 20-fold dilution in the fifth step of the flowchart of Fig. 2.
{Fig. 10}
   The figure shows ultrasound images of a modified contrast medium of Fig. 1 (a) before culture and (b) after culture, which shows specific binding ability to cancer cells.
{Fig. 11}
   The figure shows (a) a confocal fluorescence microscope image, (b) a bright-field image and (c) a superimposed image all showing specific uptake of the contrast medium of Fig. 1 by HCT116 cells (three hours after culture); and (d) a confocal fluorescence microscope image, (e) a bright-field image and (f) a superimposed image all showing a contrast medium of Comparative Example having no CCA1 antibody.
{Fig. 12}
   The figure shows (a) a confocal fluorescence microscope image, (b) a bright-field image and (c) a superimposed image all showing specific uptake of the contrast medium of Fig. 1 by AGS cells (three hours after culture); and (d) a confocal fluorescence microscope image, (e) a bright-field image and (f) a superimposed image all showing a contrast medium of Comparative Example having no CCA1 antibody.
{Fig. 13}
   The figure shows flow-cytometric analysis results showing specific uptake of the contrast medium of Fig. 1 by HCT116 cells and AGS cells (twelve hours after culture), (a) relationship between amount of fluorescence and the number of cells due to uptake of phase changeable nano liquid drop by HCT116 cell, (b) (a) the mean fluorescence of each of the cases of graph (a), (c) relationship between amount of fluorescence and the number of cells due to uptake of phase changeable nano liquid drops by AGS cells and (d) the mean fluorescence of each of the cases of graph (c).
{Fig. 14}
   The figure schematically shows a kit for preparing a contrast medium according to an embodiment of the present invention.

### {Description of Embodiments}

Contrast medium 1 according to a first embodiment of the present invention and a method for preparing the contrast medium will be described below with reference to the drawings.

The contrast medium 1 according to the embodiment has an ultrasound imageable substrate 2, at least one molecular probe (3 to 5) and at least one optical probe 6, which modify a surface of the substrate 2, as shown in Fig. 1.

The substrate 2 is constituted of a liposome, which contains perfluoro hexane therein as an inclusive and has polyethylene glycol in the surface. Perfluoro hexane is a water insoluble substance, which is phase changeable into a gaseous material by irradiation with ultrasound at an intensity of a predetermined threshold or more. In other words, perfluoro hexane produces an ultrasound imaging function by changing a phase into gaseous material.

The molecular probes 3 to 5 include a first molecular probe 3 for conjugation to be attached to the surface of the substrate 2 as a label, a second molecular probe 4 to be attached to an optical probe 6 as a label and a third molecular probe 5 for targeting to be attached to the first molecular probe 3.

As the first molecular probe 3, for example, biotin is used.

As the second molecular probe 4, for example, streptavidin is used.

As the third molecular probe 5, for example, CCA1 antibody is used.

As the optical probe 6, for example, Alexa Fluor (AF) 647 is used.

A method for preparing the contrast medium 1 according to the embodiment having the constitution as mentioned above will be described below.

The method for preparing the contrast medium 1 of the embodiment, as shown in Fig. 2, includes a first step S1 of producing a first solution containing the substrate 2, the surface of which is labeled with a first molecular probe 3; a second step S2 of producing a second solution containing the second molecular probe 4 labeled with the optical probe 6; a third step S3 of producing a third solution containing the third molecular probe 5 labeled with the first molecular probe 3; a fourth step S4 of producing a fourth solution by mixing the second solution produced in the second step and the third solution produced in the third step; a fifth step S5 of mixing and reacting the first solution produced in the first step S1 with the fourth solution produced in the fourth step S4; and a sixth step S6 of centrifuging the solution mixture produced in the fifth step S5.

To describe more specifically, in the first step S1, a first solution containing biotinylated nano liquid drops (biotin 3 is attached to the surface of the substrate 2 as a label) is produced. In the second step S2, a second solution containing streptavidin 4 labeled with AF647 6 is produced. In the third step S3, a third solution containing biotinylated CCA1 antibody prepared by labelling CCA1 antibody 5 with biotin 3 is produced.

In the fourth step S4, the fourth solution is produced by mixing the second solution (for example, 0.4 µm) and the third solution (for example, 0.4 µm) and allowing the resultant solution to stand still for 15 minutes for conjugation. In the fifth step S5, a dilution (for example, 20-fold) of the first solution (for example, 50 µL) is mixed with the fourth solution (for example, 50 µL) for a reaction, for example, at 4°C for 30 minutes. In this manner, the surface of the substrate 2 of a contrast medium is simultaneously modified with CCA1 antibody and AF647 6.

In the sixth step S6, the solution mixture produced in the fifth step S5 is centrifuged for example, at 3000G for 5 minutes. In this manner, the contrast medium 1 according to the embodiment is produced. In this step, a free composite not attached to the contrast medium 1 is separated and removed from the contrast medium.

Herein, it is important to carry out the third step S3 prior to the fourth step S4; mix the second solution and the third solution in a molar ratio of 1:1 in the fourth step S4; and mix a 10-fold or more dilution of the first solution with the fourth solution in the fifth step S5.

The concentration of the second solution can be roughly calculated, for example, by an absorption method. Furthermore, the concentration of the third solution can be accurately measured, for example, by use of an absorption method and Bradford method.

The action of the contrast medium 1 thus produced according to the embodiment will be described below.

The specific binding property of the contrast medium 1 according to the embodiment to large intestine cancer cells cultured in a Petri dish (DLD-1) was checked.

Herein, the supernatant centrifuged in the sixth step S6 was removed and the precipitate was resuspended in PBS (for example, 100 µL). The resultant solution was sprinkled on the cells and allowed to stand still for 30 minutes. The surface of the cells was washed with the culture solution.

The specific binding property was checked by a confocal fluorescence microscope and a flow-cytometer.

Confocal fluorescence microscope images are separately shown in Fig. 3 (a), (d) and Fig. 4 (a), (d) and microscopic images in the bright-field are shown in Fig. 3 (b), (e) and Fig. 4 (b), (e). The superimposed images of these are separately shown in Fig. 3 (c), (f) and Fig. 4 (c), (f). Furthermore, flow-cytometric analysis results are shown in Figs. 5 (a) to (d).

Fig. 5 (a) shows the relationship between amount of fluorescence and the number of cells due to uptake, in which (0) intact, (1) phase changeable nano liquid drops (PCND) alone, (2) no CCA1 antibody and (3) the contrast medium 1 of the embodiment; and Fig. 5 (b) shows the mean fluorescence of each of the cases of graph (a).

Fig. 5 (c) shows the relationship between amount of fluorescence and the number of cells due to uptake, in which (0) intact, (2) no CCA1 antibody and (3) the contrast medium 1 of the embodiment, when phase changeable nano liquid drops containing a different component from the case (a) is used; and Fig. 5 (d) shows the mean fluorescence of each of the cases of graph (c).

According to these figures, it is demonstrated that the contrast medium 1 according to the embodiment has a high accumulation level in the cell since it is labeled with CCA1 antibody serving as the third molecular probe 5. Since the contrast medium 1 according to the embodiment can be formed in the nano order, the contrast medium is bound to the cell surface if the culture time is short, whereas, the contrast medium is incorporated in the cell if the culture time is long. This phenomenon is called internalization. As a result, different functions can be shown as images with the passage of time from administration to a living body. Furthermore, the effect does not vary depending upon the component contained therein.

Furthermore, the contrast medium 1 according to the embodiment contains water insoluble perfluoro hexane therein as an inclusion. The perfluoro hexane is a substance, which causes phase change into a gaseous material by irradiation with ultrasound at an intensity of a predetermined threshold or more. The contrast medium 1 according to the embodiment labeled with an antibody and a pigment was subjected to functional evaluation as an ultrasound contrast medium, more specifically, evaluation of phase change from liquid to gas. The results will be described with reference to Fig. 6 and Fig. 7.

Fig. 6 shows the experiment system for ultrasound irradiation and imaging. In the figure, reference symbol 7 denotes a water vessel, reference symbol 8 a focused ultrasound transducer, reference symbol 9 an ultrasound probe for diagnosis, reference symbol 10 an amplifier, reference symbol 11 a wave generator, and reference symbol 12 ultrasonic diagnostic equipment. Furthermore, reference symbol A denotes a sample and reference symbol B degassed water. Now, the procedure for the experiment will be described below.

First, the water vessel 7 is filled with degassed water B of 37°C.

Then, a phantom (sample A) of a contrast medium according to the embodiment, which is contained in 8% acrylamide gel such that perfluoro pentane is contained in a concentration of 0.02 mg/mL, is placed in the water vessel 7. Pulse ultrasound of 3 MHz (duty ratio: 0.01, 1000 cycle) is applied to the phantom by use of the focused ultrasound transducer 8 for one second.

The ultrasound transducer 8 is driven by the wave generator 11 and the amplifier 10. During ultrasound irradiation by the ultrasound transducer 8, an ultrasound image of phantom A is obtained by an ultrasound probe 12 for diagnosis connected to the ultrasonic diagnostic equipment 12.

Figs. 7 (a) and (b) show ultrasound images of a tumor before and after irradiation with ultrasound at at least an ultrasound intensity required for changing brightness of an ultrasound image (threshold: corresponding to ultrasound intensity required for generating micron-size air bubbles for diagnosis) (see the arrow in Fig. 7 (b)). Since ultrasound for diagnosis herein has previously set to be lower than the threshold at which a phase change occurs, an echo signal obtained from the ultrasound for diagnosis is scattered wave derived from bubbles already generated.

Note that even if the ultrasound irradiation condition from the focused ultrasound transducer 8 is changed to 9 MHz, the same results as in Fig. 7 were successfully obtained by optimizing the intensity, the number of cycles and application time. From the results shown herein, the effect of the contrast medium 1 containing a water insoluble substance causing phase change from liquid to gas like the embodiment as an inclusive, is clearly demonstrated based on brightness change in the ultrasound image for diagnosis.

Note that the same effect as in the embodiment was obtained in the case of a contrast medium using a low-boiling point water-insoluble substance in combination with a high-boiling point water-insoluble substance.

As described above, it was confirmed that even if an optical probe 6 and a molecular probe 5 such as CCA1 antibody are mounted on the surface of the substrate 2, vaporization can be made.

If a contract medium is prepared in accordance with the procedure as shown in Fig. 8 (a), which is a different procedure from that of the preparation method of the embodiment, the contrast medium is agglomerated as shown in Figs. 8 (b) and (c), with the result that it becomes difficult for the contrast medium to specifically bind to a target molecule. In the procedure shown in Fig. 8 (a), after the first step S1 and the second step S2 mentioned above, the first solution and the second solution are mixed, and the resultant solution is mixed with a solution containing a biotinylated CCA1 antibody which is prepared by labeling a CCA1 antibody with biotin.

Fig. 8 (b) is a microscope photograph of cells to which a solution containing biotinylated phase changeable nano liquid drops of a contrast medium modified with streptavidin labeled with AF647 is applied; whereas Fig. 8 (c) is a microscope photograph of cells to which a solution, which is prepared by adding a solution modified with a biotinylated CCA1 antibody to the solution of Fig. 8 (b), is applied.

In the fifth step S5, if the first solution produced in the first step S1 is directly used without being diluted, a contrast medium is also agglomerated, as shown in Fig. 9 (a), with the result that it becomes difficult for the contrast medium to specifically bind to a target molecule.

As described above, the contrast medium 1 and the preparation method for the contrast medium 1 according to the embodiment has an advantage in that the contrast medium is allowed to be specifically bound only to a target molecule of a living body (cell) by means of the third molecular probe 5 without agglomerating particles of the contrast medium 1; and also has another advantage in that imaging of the state (function) of a living body, which varies with time after administration of the contrast medium, can be made by light and ultrasound.

As a result, using the fluorescence imaging by the contrast medium 1 according to the embodiment, it is possible to previously understand behavior of the contrast medium 1 according to the embodiment in the cell such as timing of internalization. It is also possible to perform ultrasound irradiation at an optimal time while observing fluorescence in real time.

To be more specific, it is possible to provide a contrast medium having sensitivity to not only ultrasound but also light, which is excellent in real-time imaging and resolution, and having a size sufficient to travel into tissues through blood vessel by labeling the ultrasound imageable substrate 2 with the molecular probe 3 and the optical probe 6. Owing to this, the contrast medium has an advantage of early diagnosing a disease at a cellular level.

It is also possible to appropriately set the timing for vaporizing the contrast medium by ultrasound irradiation. More specifically, imaging of various functions can be made also by ultrasound irradiation if the contrast medium 1 according to the embodiment is used.

In the embodiment, perfluoro hexane, which is a water insoluble substance vaporizable by ultrasound irradiation, is used as an inclusion in the contrast medium 1; however, other water insoluble substances may be used, or alternatively, water insoluble substances different in boiling point may be used in combination. Besides this, contrast substances sensitive to other modality means may be contained in the contrast medium in order to use other modality means except ultrasound and light, in combination.

The molecular probes 3 to 5 and the optical probe 6 are not limited to those used in the embodiment.

In the embodiment, a nano-size bubble liposome, which is prepared by using a solution containing perfluoro butane as an inclusion in place of perfluoro hexane and lipid membrane formed of polyethylene glycol as an outer coat, may be employed.

When the contrast medium 1 thus prepared is injected to a mouse tail, the contrast medium can be selectively accumulated only to a tumor tissue, as shown in Figs. 10 (a) and (b) due to the EPR effect due to the nano size and active binding by an antibody. Owing to this, ultrasound imaging can be advantageously made without vaporizing the inclusion.

Examples of the inclusion of the contrast medium 1 may include air and gaseous materials such as argon, perfluoro butane and perfluoro propane. The material for the outer coat is not limited to the lipid membrane formed of polyethylene glycol and lipid materials having other hydrophilic groups may be used. Furthermore, if information on internalization is not required, a step of labeling the contrast medium with an optical dye may be skipped.

Furthermore, since the contrast medium according to the embodiment has tissue selectivity to a cancer cell and an internalization ability to enter into the cell, the contrast medium can serve as a delivery means for any cancer cell. The structure of the delivery means may be the same as that of the contrast medium according to the embodiment. The delivery means may be, for example, a substrate binding to a targeting molecular probe via a conjugate molecular probe having optical activity.

The substrate may contain a desired substance having an anti-cancer function. Alternatively, a substance, which is activated only upon application of therapeutic-purpose ultrasound, may be contained. The delivery means thus constituted may be used, for example, as diagnostic/therapeutic agent.

Now, the method for preparing a contrast medium according to a second embodiment of the present invention will be described below with reference to the drawings.

The contrast medium according to the embodiment employs a mixture of two types of gaseous materials as an inclusion, more specifically, a gas mixture of perfluoro hexane and perfluoro pentane contained in a ratio of 1:1.

The size of the contrast medium according to the embodiment thus constituted is compared to those of the contrast mediums obtained by other preparation methods as shown in Table 1. Table 1 shows the results of two experimental results.

**{Table 1}**

| (1) | | | | | | | | | | | | |
|---|---|---|---|---|---|---|---|---|---|---|---|---|
| | Measurement results (pixels) | | | | | | | | | | Average (µm) | Standard deviation (µm) |
| First solution | 13 | 12 | 13 | 12 | 13 | 11 | 11 | 12 | 11 | 13 | 0.756 | 0.055 |
| Single addition of SA | 14 | 11 | 25 | 59 | 24 | 113 | 110 | 27 | 39 | 11 | 2.706 | 2.420 |
| Embodiment | 10 | 12 | 12 | 13 | 13 | 14 | 13 | 14 | 19 | 11 | 0.819 | 0.152 |
| | | | | | | | | | | | | |

| (2) | | | | | | | | | | | | |
|---|---|---|---|---|---|---|---|---|---|---|---|---|
| First solution | 14 | 12 | 12 | 15 | 12 | 13 | 16 | 11 | 13 | 11 | 0.806 | 0.104 |
| Single addition of SA | 23 | 61 | 79 | 12 | 14 | 13 | 14 | 62 | 85 | 68 | 2.694 | 1.899 |
| Embodiment | 14 | 11 | 11 | 15 | 12 | 12 | 13 | 10 | 14 | 15 | 0.794 | 0.110 |

If streptavidin (SA) is singly added to the first solution produced in the first step, the size of the contrast medium considerably increases and agglomerated; however, if the contrast medium is prepared by the preparation method according to the embodiment, the medium can be labeled with an antibody while maintaining a nano-size. The mixing ratio of the water insoluble substances is not limited to those of the embodiment. The type of inclusions and combination and mixing ratio thereof, can be appropriately changed depending upon the vaporization conditions (size of air bubbles, retention time of vapor phase, frequency responsiveness and ultrasound intensity threshold).

As described above, the contrast medium produced by the preparation method according to the embodiment, even if a gaseous material serving as an inclusion is changed, causes no agglomeration of particles of the contrast medium and specifically bind only to a target molecule of a living body (cell) by means of the third molecular probe. In addition, imaging of the state (function) of a living body, which varies with time after administration of the contrast medium, can be made.

Herein, specific binding property to HCT116 cell and AGS cell in place of DLD1 cell was checked, without changing CCA1 antibody serving as the third molecular probe 5.

To describe it more specifically, the contrast medium was added to culture dishes of HCT116 cell and AGS cell and allowed to stand still, for example, one hour, and the surface are washed with the culture medium. For example, three hours later, cell-specific uptake was observed by a confocal laser microscope. The cells were cultured for 12 hours, washed with PBS, removed from the culture dishes with trypsin/EDTA and subjected to flow-cytometric analysis.

Accumulation results are shown in Figs. 11 (a) to (f) and Figs. 12 (a) to (f). Figs. 11 (a) to (c) and Figs. 12 (a) to (c) show HCT116 cell-specific uptake; whereas Figs. 11 (d) to (f) and Figs. 12 (d) to (f) show AGS cell-specific uptake. The flow-cytometric results are shown in Figs. 13 (a) to (d).

The contrast medium according to the embodiment was also accumulated in HCT116 cell and travels into the cell; however, the contrast medium was not accumulated in AGS cell, whose expression level of CCA1 is reported to be low.

As described above, the contrast medium according to the embodiment can specifically bind to the antigen corresponding to the antibody employed as the label and advantageously distinguish the cell from other cells.

In the present invention, the contrast medium 1 can be produced by a preparation kit 21 shown in Fig. 14.

The preparation kit 21 has storage containers 22, 23 and 24 for aseptically storing first to third solutions, respectively, and channels 25 and 26 connecting between these storage containers 22 to 24. The channels 25, 26 are equipped respectively with valves 27 and 28, which are operated to open and close the channels 25 and 26.

The second solution and the third solution are mixed by opening the valve 28 of the second channel 26 to produce the fourth solution, and then the first solution and the fourth solution are mixed by opening the valve 27 of the first channel 25. The contrast medium 1 according to the embodiment can be aseptically produced by setting the whole preparation kit in a centrifuge. Note that a safety apparatus may be provided such that the valve 28 and valve 27 are opened only in this order.

### {Reference Signs List}

- 1: Contrast medium
- 2: Substrate
- 3: First molecular probe
- 4: Second molecular probe
- 5: Third molecular probe
- 6: Optical probe
- 21: Preparation kit
- 22 to 24: Storage containers
- 25, 26: Channels

## Claims

1. A contrast medium comprising at least one molecular probe and at least one optical probe on a surface of a substrate having an ultrasound imaging function.

2. The contrast medium according to Claim 1, comprising a first molecular probe, an optical probe labeled with a second molecular probe and a third molecular probe capable of binding to a cell or a tissue in a disease-specific manner.

3. The contrast medium according to Claim 2, wherein the third molecular probe is labeled with the first molecular probe.

4. The contrast medium according to Claim 2 or Claim 3, wherein the second molecular probe and the third molecular probe are contained in a molar ratio of 1:1.

5. The contrast medium according to any one of Claims 1 to 4, wherein the substrate is a capsule-type contrast medium comprising a contrast substance having an imaging function included in an outer coat formed of lipid membrane.

6. The contrast medium according to Claim 5, wherein the contrast substance is a water insoluble substance vaporizable by ultrasound irradiation.

7. The contrast medium according to Claim 5, wherein the contrast substance is a gaseous material.

8. A method for preparing the contrast medium according to Claim 2, comprising:
a first step of producing a first solution containing the substrate of which the surface is labeled with the first molecular probe;
a second step of producing a second solution containing the second molecular probe labeled with the optical probe;
a third step of producing a third solution containing the third molecular probe labeled with the first molecular probe;
a fourth step of producing a fourth solution by mixing the second solution produced in the second step and the third solution produced in the third step;
a fifth step of mixing and reacting the first solution produced in the first step with the fourth solution produced in the fourth step; and
a sixth step of centrifuging the solution mixture produced in the fifth step.

9. The preparation method according to Claim 8, wherein the second molecular probe contained in the second solution and the third molecular probe contained in the third solution to be mixed in the fourth step are present in a molar ratio of 1:1.

10. The preparation method according to Claim 8 or 9, wherein in the fifth step, a 10-fold or more dilution of the first solution produced in the first step is mixed.

11. A kit for preparing the contrast medium according to Claim 2, comprising: a plurality of storage containers separately storing a first solution containing the substrate of which a surface is labeled with the first molecular probe, a second solution containing the second molecular probe labeled with the optical probe, and a third solution containing the third molecular probe labeled with the first molecular probe; and openable and closable channels connected with the storage containers.
